# EUROPEAN PATENT SPECIFICATION

(11) **EP 3 496 819 B1**
(45) Date of publication and mention of the grant of the patent: **29.07.2020**
(21) Application number: 17745753.8
(22) Date of filing: 03.08.2017
(51) Int. Cl.: A61Q 7/00, A61K 8/19, A61K 8/20

(54) **HAIR TREATMENT COMPOSITION**
HAARBEHANDLUNGSMITTEL
COMPOSITION POUR LE TRAITEMENT DES CHEVEUX

(30) Priority: 12.08.2016 EP 16183912
(43) Date of publication of application: 19.06.2019
(73) Proprietor: Unilever N.V., 3013 AL Rotterdam (NL); Unilever PLC, London, Greater London EC4Y 0DY (GB)
(72) Inventor: DAS, Somnath, Whitefield Bangalore 560 066 (IN); GADGIL, Vijay, Ramchandra, Thubharahalli Bengaluru 560 066 Karnataka (IN); GHOSH, Debjani, Whitefield Bangalore 560 066 (IN); JAGADISH, Nidhi, Whitefield Bangalore 560 066 (IN)
(74) Representative: Chisem, Janet
(86) International application number: PCT/EP2017/069676
(87) International publication number: WO 2018/029080

(56) References cited:
- EP-A1- 2 098 218
- EP-A1- 2 883 573
- JP-A- 2006 328 004
- WRAY AND DANIELS: "Precipitation of Calcite and Aragonite", JOURNAL OF THE AMERICAN CHEMICAL SOCIETY, vol. 79, no. 9, 7 May 1957 (1957-05-07), pages 2031-2034, XP002761770, DOI: 10.1021/ja01566a001

## Description

### Field of the invention

The present invention relates to hair treatment compositions. In particular, the present invention relates to hair treatment compositions that stimulate hair growth.

### Background of the invention

Various compounds and combinations thereof are known for use in the treatment of hair to stimulate hair growth.

JP2006328004 (Kawahara) discloses a method to provide a hair tonic which contains weathering reef-producing coral as a raw material and has a high hair-growing effect. This method for producing the hair tonic is characterized by comprising a process for heating the weathering reef-producing coral at, for example, 898 to 1500 °C to chemically change calcium carbonate (CaCO₃) contained in the weathering reef-producing coral into calcium oxide (CaO) and a process for chemically changing the calcium oxide (CaO) into calcium hydroxide [Ca(OH)₂].

JP10130127 (Nakajima) relates to a hair tonic which comprises calcium phosphate powder in the extract extracted from seaweed. The object of the invention is to the hair tonic of this document stimulates the activity of the skin cells of a hair root and the scalp, and promotes hair fostering while clearing the pores.

WO14173635A1 (Unilever) discloses a topical composition for hair growth comprising synergistic combination of extracts of plant source. The composition not only retains hair bulbs in the anagen state for longer time but also ensures longer length of hair. This is achieved through a composition, which comprises a gallic acid ester and an extract of *Glycyrrhiza glabra* or *Sesamum indicum* which is applied on the hair or scalp for the desired benefits.

In a scientific article published in Journal Of The American Chemical Society vol. 79, no. 9, 7 May 1957 (1957-05-07), pages 2031-2034, the authors, Wray et al, have disclosed precipitation of two common polymorphs of calcium carbonate, calcite and aragonite by mixing soluble carbonate solutions with solutions of calcium ions under conditions of controlled temperature, concentration and aging. Results from these experiments provide new information regarding the factors affecting the formation of the two crystalline varieties.

Although the hair treatment compositions in prior art provide for hair growth, there is need for improved and superior compositions.

It is therefore an object of the present invention to provide improved and superior compositions for hair growth.

Surprisingly, it has been found that a polymorph of calcium carbonate and a water-soluble compound of calcium work synergistically to stimulate hair growth.

### Summary of the invention

Accordingly, in a first aspect, the present invention provides a hair treatment composition comprising:
(i) a polymorph of calcium carbonate;
(ii) a water-soluble compound of calcium; and
(iii) a suitable carrier
wherein said polymorph is at least one of calcite, vaterite or aragonite and wherein particle size of said polymorph is 1 µm to 10 µm.

In a second aspect the present invention provides a method of stimulating hair growth comprising, in sequence, the steps of:
(i) applying a composition of the first aspect onto the scalp;
(ii) rinsing off said composition with water, if necessary, and
(iii) allowing the scalp to dry.

In a third aspect the present invention provides the use of a polymorph of calcium carbonate for stimulating hair growth.

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of' or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

### Detailed description of the invention

Hair are filamentous, keratinized structures consisting of a shaft and a root. The shaft is composed of specialized keratinocytes. The root lies within the hair follicle and comprises the germinative matrix and the keratogenous zone.

There has been continuous effort to find a safe, reliable method of minimizing hair loss or increasing hair growth and thus reducing baldness to various degrees. Though hair loss is not a disease, it impacts the persons concerned psychologically and seriously affects the individual's self-esteem. The cause for the problem of hair loss is not always the same in all. There are several factors which influence the process by which hair grows and there are many contributory factors that can alter the normal vigorous growth of hair.

The hair growth cycle is divided into three phases. The first is anagen phase in which the hair is growing actively, with a very substantial level of cell proliferation occurring in the hair follicle. It is followed by a catagen phase, when the follicle slows down its proliferative activity temporarily to permit hair development. The final phase is telogen phase, in which the follicle simply stops growing and regresses, until the hair is shed to begin a new anagen phase.

It is natural to lose some hair on a daily basis as this is a part of the hair renewal process. This cycling process of hair growth continues throughout the life of an individual. But when this loss becomes excessive it becomes a problem which people try to solve by the use of cosmetic formulations. As appearance and looks are an important aspect in society, hair loss is considered a major problem. Millions of people worldwide suffer from hair loss and are keen to find a solution to treat such hair loss. Various hair growth products and treatments have been developed to solve such a problem but the problem still persists.

In a first aspect the invention provides a hair treatment composition comprising:
(i) a polymorph of calcium carbonate;
(ii) a water-soluble compound of calcium; and
(iii) a suitable carrier
wherein said polymorph is at least one of calcite, vaterite or aragonite and wherein particle size of said polymorph is 1 µm to 10 µm.

### The polymorph

In the context of crystallography, polymorphism is the condition in which a solid chemical compound exists in more than one crystalline form. The forms differ somewhat in physical and sometimes in chemical properties.

The polymorphic forms of calcium carbonate are described for example, in an article published in Surf Interface Anal. 2008 October; 40 (10): 1356-1361. The article discloses three polymorphs of anhydrous calcium carbonate: vaterite, aragonite and calcite. Aragonite and calcite are more thermodynamically stable structures, and they most commonly occur in nature. For example, the abalone shell is comprised of calcite (prismatic layer) and aragonite (nacreous layer). Vaterite, also known as µ-CaCO3, less commonly occurs in nature because it is the least thermodynamically stable polymorph. Vaterite can rapidly transform to calcite and aragonite in aqueous solution.

In the compositions according to the invention, the polymorph is at least one of calcite, vaterite or aragonite. Calcite and vaterite are particularly preferred. Calcite is most preferred because it provides superior efficacy and stability.

The particle size of the polymorph is 1 µm to 10 µm. More preferably the particle size of the polymorph is 2.5 µm to 8 µm and further more preferably it is 5 µm to 7 µm.

Preferably, in the compositions according to the invention, the amount of the polymorph is 0.005 to 10 % by weight. More preferably, it is 2 to 8 % by weight and most preferably it is 3 to 5 % by weight of the composition.

### Water-soluble compound of calcium

The water-soluble compound of calcium interacts synergistically with the polymorph to stimulate hair growth.

It is preferred that the compositions in accordance with the invention comprise 0.1 to 30 % by weight of the water-soluble compound. More preferably the compositions comprise 1 to 10 % by weight.

Preferably the water-soluble compound of calcium is at least one of a calcium halide such calcium chloride, calcium bromide or calcium iodide or another compound of calcium which is at least one of calcium nitrate, calcium acetate, calcium azide, calcium chlorate, calcium bromate, calcium iodate, calcium perchlorate, or calcium permanganate. It is particularly preferred that the water-soluble compound of calcium is calcium chloride and calcium nitrate.

### Carrier

The term hair treatment composition, as used herein, is meant to include a composition for topical application to hair or scalp of mammals, especially humans. By topical is meant that the composition is applied to the external surface of the body. In the present invention, this is achieved by applying the composition to the hair or scalp. Compositions for achieving the desired benefits by way of ingestion into the human body are excluded from the scope of the present invention. Such a composition may be generally classified as leave-on or rinse off, and includes any product applied to a human body for improving the appearance, cleansing, odour control or general aesthetics of scalp and hair. The composition of the present invention can be in the form of a liquid, lotion, cream, foam, scrub, gel, shampoo, conditioner, shower gel or bar, hair styling cream, hair spray, hair styling gel, or a hair oil. The composition of the present invention is preferably a leave-on composition which is meant to be left in contact with the scalp for at least 3 minute post application and not washed or rinsed with water immediately after application.

The carrier serves to carry or convey the essential, preferred and optional ingredients of the compositions. A carrier would include water, saline water, brine, oils selected from triglycerides, silicones, paraffin and wax esters, and aerosol propellants.

### Optional ingredients

Optional ingredients that can be used in the composition of the present invention include silicones, dolomite, hydrotalcite, silica, porous clays, polymers as structurants, surfactants, emollients and viscosity modifiers.

Hair treatment compositions according to the invention may suitably take the form of shampoos, conditioners, sprays, mousses, gels, waxes, hair-oil or lotions.

Particularly preferred product forms are shampoos, post-wash conditioners (leave-in and rinse-off) and hair treatment products such as hair oils and lotions.

Shampoo compositions are generally aqueous, i.e. they have water or an aqueous solution or a lyotropic liquid crystalline phase as their major component.

Suitably, the shampoo composition will comprise from 50 to 98 %, preferably from 60 to 90 % water by weight based on the total weight of the composition.

Shampoo compositions according to the invention will generally comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic cleansing surfactants are sodium lauryl sulphate, sodium lauryl ether sulphate (n)EO, (where n is from 1 to 3), sodium lauryl ether sulphosuccinate(n)EO, (where n is from 1 to 3), ammonium lauryl sulphate, ammonium lauryl ether sulphate(n)EO, (where n is from 1 to 3), sodium cocoyl isethionate and lauryl ether carboxylic acid (n) EO (where n is from 10 to 20).

Mixtures of any of the foregoing anionic cleansing surfactants may also be suitable.

The total amount of anionic cleansing surfactant in shampoo compositions of the invention generally ranges from 0.5 to 45 %, preferably from 1.5 to 35 %, more preferably from 5 to 20 % by weight anionic cleansing surfactant based on the total weight of the composition.

Optionally, a shampoo composition of the invention may contain further ingredients as described below to enhance performance and/or consumer acceptability.

The composition can include co-surfactants, to help impart aesthetic, physical or cleansing properties to the composition.

An example of a co-surfactant is a nonionic surfactant, which can be included in an amount ranging from 0.5 to 8%, preferably from 2 to 5% by weight based on the total weight of the composition.

For example, representative nonionic surfactants that can be included in shampoo compositions of the invention include condensation products of aliphatic (C₈ - C₁₈) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or di-ethanolamide and coco mono-isopropanolamide. Further nonionic surfactants which could be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula: RO - (G)ₙ, wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix® NS10 ex Seppic; Plantaren® 1200 and Plantaren® 2000 ex Henkel.

Other sugar-derived nonionic surfactants which can be included in compositions of the invention include the C₁₀-C₁₈ N-alkyl (C₁-C₆) polyhydroxy fatty acid amides, such as the C₁₂-C₁₈ N-methyl glucamides, as described for example in
WO9206154 A1 and US5194639 B, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.5 to about 8%, preferably from 1 to 4% by weight based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

The total amount of surfactant (including any co-surfactant, and/or any emulsifier) in a shampoo composition of the invention is generally from 1 to 50 %, preferably from 2 to 40 %, more preferably from 10 to 25 % by total weight surfactant based on the total weight of the composition.

Cationic polymers are preferred ingredients in a shampoo composition of the invention for enhancing conditioning performance.

Suitable cationic polymers may be homopolymers which are cationically substituted or may be formed from two or more types of monomers. The weight average (M_{w}) molecular weight of the polymers will generally be between 100 000 and 2 M Daltons. The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerised in the amine form and then converted to ammonium by quaternisation.

The cationic polymers can comprise mixtures of monomer units derived from amine- and/or quaternary ammonium-substituted monomer and/or compatible spacer monomers.

Suitable cationic polymers include, for example:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms,; and,
- cationic polyacrylamides.

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula: A-O-[R-N⁺(R¹)(R²)(R³)X⁻], wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200®.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR® trademark series). Examples of such materials are JAGUAR® C13S, JAGUAR® C14, JAGUAR® C15 and JAGUAR® C17.

Mixtures of any of the above cationic polymers may be used.

Cationic polymer will generally be present in a shampoo composition of the invention at levels of from 0.01 to 5%, preferably from 0.05 to 1%, more preferably from 0.08 to 0.5% by total weight of cationic polymer based on the total weight of the composition.

Preferably an aqueous shampoo composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol® 420, Carbopol® 488 or Carbopol® 493. Polymers of acrylic acid cross-linked with a polyfunctional agent may also be used; they are available commercially as Carbopol® 910, Carbopol® 934, Carbopol® 941 and Carbopol® 980. An example of a suitable copolymer of a carboxylic acid containing monomer and acrylic acid esters is Carbopol® 1342. All Carbopol® materials are available from Goodrich.

Suitable cross-linked polymers of acrylic acid and acrylate esters are Pemulen® TR1 or Pemulen® TR2. A suitable heteropolysaccharide gum is xanthan gum, for example that available as Kelzan® mu.

Mixtures of any of the above suspending agents may be used. Preferred is a mixture of cross-linked polymer of acrylic acid and crystalline long chain acyl derivative.

Suspending agent will generally be present in a shampoo composition of the invention at levels of from 0.1 to 10%, preferably from 0.5 to 6%, more preferably from 0.9 to 4% by total weight of suspending agent based on the total weight of the composition.

Conditioner compositions will typically comprise one or more cationic conditioning surfactants which are cosmetically acceptable and suitable for topical application to the hair.

Preferably, the cationic conditioning surfactants have the formula N⁺(R¹)(R²)(R³)(R⁴), wherein R¹, R², R³ and R⁴ are independently (C₁ to C₃₀) alkyl or benzyl.

Preferably, one, two or three of R¹, R², R³ and R⁴ are independently (C₄ to C₃₀) alkyl and the other R¹, R², R³ and R⁴ group or groups are (C₁-C₆) alkyl or benzyl.

More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (e.g., oleyl). Alkyl groups are optionally ethoxylated on the alkyl chain with one or more ethyleneoxy groups.

Suitable cationic conditioning surfactants for use in conditioner compositions according to the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (e.g., Arquad® 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners according to the invention is cetyltrimethylammonium chloride, available commercially, for example as GENAMIN® CTAC, ex Hoechst Celanese. Another particularly useful cationic surfactant for use in conditioners according to the invention is behenyltrimethylammonium chloride, available commercially, for example as GENAMIN® KDMP, ex Clariant.

Another example of a class of suitable cationic conditioning surfactants for use in the invention, either alone or in admixture with one or more other cationic conditioning surfactants, is a combination of (i) and (ii) below:
(i) an amidoamine corresponding to the general formula (I): 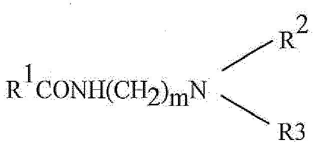
   in which R¹ is a hydrocarbyl chain having 10 or more carbon atoms,
   R² and R³ are independently selected from hydrocarbyl chains of from 1 to 10 carbon atoms, and
   m is an integer from 1 to about 10; and
(ii) an acid.

As used herein, the term hydrocarbyl chain means an alkyl or alkenyl chain.

Preferred amidoamine compounds are those corresponding to formula (I) in which
R¹ is a hydrocarbyl residue having from about 11 to about 24 carbon atoms,
R² and R³ are each independently hydrocarbyl residues, preferably alkyl groups, having from 1 to about 4 carbon atoms, and
m is an integer from 1 to about 4.

Preferably, R² and R³ are methyl or ethyl groups.

Preferably, m is 2 or 3, i.e. an ethylene or propylene group.
Preferred amidoamines useful herein include stearamido-propyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylmine, behenamidoethyldiethylamine, behenamidoethyldimethylamine, arachidamidopropyldimethylamine, arachidamidopropyidiethylamine, arachid-amidoethyldiethylamine, arachidamidoethyldimethylamine, and mixtures thereof.
Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE® S-13 available from Inolex (Philadelphia Pennsylvania, USA) and AMIDOAMINE® MSP available from Nikko (Tokyo, Japan), stearamidoethyldiethylamine with a tradename AMIDOAMINE® S available from Nikko, behenamidopropyldimethylamine with a tradename INCROMINE® BB available from Croda (North Humberside, England), and various amidoamines with tradenames SCHERCODINE® series available from Scher (Clifton New Jersey, USA).

Acid (ii) may be any organic or mineral acid which is capable of protonating the amidoamine in the hair treatment composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate all the amidoamine present, i.e. at a level which is at least equimolar to the amount of amidoamine present in the composition.

In conditioners of the invention, the level of cationic conditioning surfactant will generally range from 0.01 to 10%, more preferably 0.05 to 7.5%, most preferably 0.1 to 5% by total weight of cationic conditioning surfactant based on the total weight of the composition.

Conditioners of the invention will typically also incorporate a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.01 to 10%, preferably from 0.1 to 8%, more preferably from 0.2 to 7 %, most preferably from 0.3 to 6 % by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

Compositions of the invention may suitably take the form of a hair oil, for pre-wash or post-wash use. Typically, hair oils will predominantly comprise water-insoluble oily conditioning materials, such as triglycerides, mineral oil and mixtures thereof.

Compositions of the invention may also take the form of a hair lotion, typically for use in between washes. Lotions are aqueous emulsions comprising water-insoluble oily conditioning materials. Suitable surfactants can also be included in lotions to improve their stability to phase separation.

Hair treatment compositions according to the invention, particularly water-based shampoos and hair conditioners, will preferably also contain one or more silicone conditioning agents.

Particularly preferred silicone conditioning agents are silicone emulsions such as those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter (D_{3,2}) in the composition of the invention ranging from 0.01 to 20 micrometer, more preferably from 0.2 to 10 micrometer.

A suitable method for measuring the Sauter mean droplet diameter (D_{3,2}) is by laser light scattering using an instrument such as a Malvern® Mastersizer.

Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter (D_{3,2}) of less than 0.15 micrometers are generally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions/microemulsions of dimethiconol. Also suitable are amodimethicone emulsions such as DC2-8177 and DC939 (from Dow Corning) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active block copolymer is according to the following formula: HO(CH₂CH₂O)ₓ(CH(CH₃)CH₂O)_{y}(CH₂CH₂O)ₓ H wherein the mean value of x is 4 or more and the mean value of y is 25 or more.

Another preferred form of the surface active block copolymer is according to the following formula: (HO(CH₂CH₂O)ₐ(CH(CH₃)CH2O)_{b})₂-N-CH₂-CH₂-N((OCH₂CH(CH₃))_{b}(OCH₂CH₂)ₐ OH)₂, wherein the mean value of a is 2 or more and the mean value of b is 6 or more.

Mixtures of any of the above described silicone emulsions may also be used.

The above described silicone emulsions will generally be present in a composition of the invention at levels of from 0.05 to 10 %, preferably 0.05 to 5 %, more preferably from 0.5 to 2 % by total weight of silicone based on the total weight of the composition.

A composition of the invention may contain other ingredients for enhancing performance and/or consumer acceptability. Such ingredients include fragrance, dyes and pigments, pH adjusting agents, pearlescers or opacifiers, viscosity modifiers, and preservatives or antimicrobials. Each of these ingredients will be present in an amount effective to accomplish its purpose. Generally these optional ingredients are included individually at a level of up to 5 % by weight of the total composition.

Hair treatment compositions of the invention are primarily intended for topical application to the hair and/or scalp of a human subject, either in rinse-off or leave-on compositions, for the treatment of dry, damaged and/or unmanageable hair.

### Method and Use

In a second aspect, the invention relates to a method of stimulating hair growth comprising, in sequence, the steps of:
(i) applying the composition of the first aspect of the invention onto the scalp;
(ii) rinsing off said composition with water, if necessary, and
(iii) allowing the scalp to dry.

The step of rinsing off is applicable, e.g., to shampoos.

It is preferred that the method is non-therapeutic (cosmetic) method. Such a method of stimulating hair growth is a cosmetic treatment, as opposed to therapeutic method, when it is done with the intention of improving bodily appearance.

In a third aspect, the invention provides the use of a polymorph of calcium carbonate for stimulating hair growth. In this case too, it is preferred that the use is non-therapeutic or cosmetic use. Alternatively, the use is for therapeutic purpose.

The invention will now be illustrated by means of the following non-limiting examples.

### Examples

### Materials

The following commercially available materials were used for the various experiments described hereinafter:
William E media W4128 (Sigma Aldrich), calcium chloride (Sigma Aldrich), calcium nitrate, sodium carbonate (Merck), hair follicles from donors who were undergoing hair transplantation surgery (after obtaining all concerned ethical clearances) and deionized water.

### Method

The hair follicle is a complex mini-organ that is home to a variety of cells like the dermal papillae, stem cells, melanocytes, keratinocytes, transit amplifying cells and many more. Each cell type has a particular role in the formation of the hair shaft. Hair follicles undergo a cyclic growth pattern which can be sub-divided into anagen (the growth phase), catagen (the regression phase), telogen (the resting phase) and exogen (the shedding phase). When the follicles are present in the anagen phase, the length of the shaft increases. This is the phase in which most of the hair growth modulators act by increasing the rate of formation of hair shaft.

### Selection of hair follicle

The in-vitro hair follicle model comprises of hair follicles as indicated earlier. Briefly, an organ culture of follicular units, consisting of two hair follicles was gently removed from the subcutaneous layer using a scalpel blade. Anagen hair follicles containing follicular units were identified by their well-rounded structure at the bulb.

**Table 1: Composition of the culture medium**

| Component | Medium g/L | * Calcium excluded media g/L |
|---|---|---|
| Calcium Chloride | 0.2 | <0.005 |
| Magnesium Sulfate (anhydrous) | 0.0977 | 0.0977 |
| Potassium Chloride | 0.4 | 0.4 |
| Sodium Bicarbonate | 2.2 | 2.2 |
| Sodium Chloride | 6.8 | 6.8 |
| Sodium Phosphate Monobasic (anhydrous) | 0.122 | 0.122 |
| Amino Acids | 0.78 | 0.78 |
| Vitamins | 0.0011 | 0.0011 |
| D-Glucose | 2 | 2 |
| Glutathione (reduced) | 0.00005 | 0.00005 |
| Methyl Linoleate | 0.00003 | 0.00003 |
| Phenol Red • Na | 0.0107 | 0.0107 |
| Pyruvic Acid • Na | 0.025 | 0.025 |

| | | |
|---|---|---|
| * In the calcium excluded media, the amount of residual soluble calcium is less than 0.005 g/L, lower than the threshold limit for showing any activity. It is experimentally improbable to lower the concentration of calcium below this limit through chemical separation means. | | |

### Evaluation of the growth of hair follicle:

Hair follicles in the Anagen phase were placed in 500 µl of the medium described above (corresponding to 36 µg soluble calcium) in a 24-well plate (single follicular unit with two follicles/ well).

For the purpose of comparison, a control experiment (outside the invention) was conducted in which there was no polymorph of calcium carbonate in the medium. In the case of the experimental sets, the medium included a polymorph (corresponding to 5 µg calcium) and mixed well. The culture medium (was changed every three days along with the test extract. For the purpose of evaluation of hair growth, images were captured every alternate day for a period of 14 days using a stereo microscope (Olympus® SZ40) with photo-capturing software.

The length of hair shaft was measured from the captured images using a software known as Image ProPlus®. The readings were recorded after comparing the lengths of the shafts on Day 0 with the length on Day 12 for each test extract and on further comparison with its corresponding control experiments (minus the polymorph).

### Example 1: Synergistic effect of water-soluble and water-insoluble calcium on hair growth

This example illustrates synergistic effect of water-soluble and water-insoluble calcium on hair growth.

**Table 2**

| Set | Culture | Polymorph and its amount /µg | | Water-soluble calcium (µg) | % increase in length of shaft w.r.t. control |
|---|---|---|---|---|---|
| A | Medium | - | - | 36 | 0 |
| B | Medium (Calcium excluded) | - | - | <1 | -86* |
| C | Medium (Control) | calcite | 5 | <1 | -63* |
| Ex 1 | Media | calcite | 5 | 36 | 56 |

| | | | | | |
|---|---|---|---|---|---|
| * Negative growth indicates control hair grew longer than experimental | | | | | |

It is evident from the data in Table 2 that presence of the water-soluble compound and the polymorph is necessary for hair growth.

### Example 2: Effect of various polymorphs

This example demonstrates the effect of different polymorphs of calcium carbonate.

**Table 3**

| Set | Culture | Polymorph and its amount /µg | | Water-soluble calcium (µg) | % increase in length of shaft w.r.t. control |
|---|---|---|---|---|---|
| A | Media | -- | | 36 | 0 |
| Ex 2 | Media | calcite | 5 | 36 | 56 |
| Ex 3 | Media | vaterite | 5 | 36 | 52 |
| Ex 4 | Media | aragonite | 5 | 36 | 28 |

The data in Table 3 makes it clear that all polymorphs of calcium carbonate act synergistically with the water-soluble compound of calcium and they all leading to hair growth.

### Example 3: Effect of particle size of polymorph

This example demonstrates the effect of particle size of the polymorph on hair growth.

**Table 4**

| Set | Culture | Polymorph and its amount /µg | | Average particle size (µm) | Water - soluble calcium (µg) | % increase in length of shaft w.r.t. control |
|---|---|---|---|---|---|---|
| A | Media | -- | -- | -- | 36 | 0 |
| Ex 5 | Media | calcite | 5 | 6 | 36 | 56 |
| Ex 6 | Media | calcite | 5 | 4 | 36 | 33 |
| Ex 7 | Media | calcite | 5 | 2.5 | 36 | 11 |

The above table shows that increase in particle size of the polymorph leads to a corresponding increase in hair growth. For best results, the particle size of the water-insoluble calcium should be more than 5µm.

## Claims

1. A hair treatment composition comprising
(i) a polymorph of calcium carbonate;
(ii) a water-soluble compound of calcium; and
(iii) a suitable carrier
wherein said polymorph is at least one of calcite, vaterite or aragonite and wherein particle size of said polymorph is 1 µm to 10 µm.

2. A composition as claimed in claim 1 wherein said particle size is 2.5 µm to 8 µm.

3. A composition as claimed in claim 1 or 2 wherein particle size of said polymorph is 5 µm to 7 µm.

4. A composition as claimed in any of claims 1 to 3 wherein said water-soluble compound of calcium is at least one of a calcium halide such calcium chloride, calcium bromide or calcium iodide or another compound of calcium which is at least one of calcium nitrate, calcium acetate, calcium azide, calcium chlorate, calcium bromate, calcium iodate, calcium perchlorate, or calcium permanganate.

5. A composition as claimed in any of claims 1 to 4 wherein said composition comprises 0.005 to 10 % by weight of said polymorph.

6. A composition as claimed in any of claims 1 to 5 wherein said composition comprises 0.01 to 30 % by weight of said water-soluble compound of calcium.

7. A non-therapeutic method of stimulating hair growth comprising, in sequence, the steps of:
(i) applying a composition as claimed in any of claims 1 to 6 onto the scalp;
(ii) rinsing off said composition with water, if necessary, and
(iii) allowing the scalp to dry.

8. Non-therapeuctic use of a polymorph of calcium carbonate for stimulating hair growth.

## Patentansprüche

1. Haarbehandlungszusammensetzung, umfassend
(i) ein Polymorph von Calciumcarbonat;
(ii) eine wasserlösliche Calciumverbindung; und
(iii) einen geeigneten Träger,
wobei das Polymorph mindestens eines von Calcit, Vaterit oder Aragonit ist und wobei die Partikelgröße des Polymorphs 1 µm bis 10 µm beträgt.

2. Zusammensetzung, wie im Anspruch 1 beansprucht, wobei die Partikelgröße 2,5 µm bis 8 µm beträgt.

3. Zusammensetzung, wie im Anspruch 1 oder 2 beansprucht, wobei die Partikelgröße des Polymorphs 5 µm bis 7 µm beträgt.

4. Zusammensetzung, wie in irgendeinem der Ansprüche 1 bis 3 beansprucht, wobei die wasserlösliche Calciumverbindung mindestens eine von Calciumhalogenid, wie Calciumchlorid, Calciumbromid oder Calciumiodid, oder eine andere Calciumverbindung ist, welche mindestens eine von Calciumnitrat, Calciumacetat, Calciumazid, Calciumchlorat, Calciumbromat, Calciumiodat, Calciumperchlorat oder Calciumpermanganat ist.

5. Zusammensetzung, wie in irgendeinem der Ansprüche 1 bis 4 beansprucht, wobei die Zusammensetzung 0,005 bis 10 Gewichts-% des Polymorphs umfasst.

6. Zusammensetzung, wie in irgendeinem der Ansprüche 1 bis 5 beansprucht, wobei die Zusammensetzung 0,01 bis 30 Gewichts-% der wasserlöslichen Calciumverbindung umfasst.

7. Nicht-therapeutisches Verfahren zum Stimulieren von Haarwuchs, umfassend, der Reihe nach, die Schritte:
(i) Auftragen einer Zusammensetzung, wie in irgendeinem der Ansprüche 1 bis 6 beansprucht, auf die Kopfhaut;
(ii) Abspülen der Zusammensetzung mit Wasser, falls erforderlich, und
(iii) Trocknenlassen der Kopfhaut.

8. Nicht-therapeutische Verwendung eines Polymorphs von Calciumcarbonat zum Stimulieren von Haarwuchs.

## Revendications

1. Composition pour le traitement de cheveux comprenant
(i) un polymorphe de carbonate de calcium ;
(ii) un composé de calcium soluble dans l'eau ; et
(iii) un support approprié
dans laquelle ledit polymorphe est au moins un de calcite, vatérite ou aragonite et dans laquelle la taille de particule dudit polymorphe est de 1 µm à 10 µm.

2. Composition selon la revendication 1, dans laquelle ladite taille de particule est de 2,5 µm à 8 µm.

3. Composition selon la revendication 1 ou 2, dans laquelle la taille de particule dudit polymorphe est de 5 µm à 7 µm.

4. Composition selon l'une quelconque des revendications 1 à 3, dans laquelle ledit composé de calcium soluble dans l'eau est au moins un d'un halogénure de calcium, tel que chlorure de calcium, bromure de calcium ou iodure de calcium ou un autre composé de calcium qui est au moins un de nitrate de calcium, acétate de calcium, azide de calcium, chlorate de calcium, bromate de calcium, iodate de calcium, perchlorate de calcium, ou permanganate de calcium.

5. Composition selon l'une quelconque des revendications 1 à 4, dans laquelle ladite composition comprend de 0,005 à 10 % en masse dudit polymorphe.

6. Composition selon l'une quelconque des revendications 1 à 5, dans laquelle ladite composition comprend de 0,01 à 30 % en masse dudit composé de calcium soluble dans l'eau.

7. Procédé non-thérapeutique de stimulation de la croissance des cheveux comprenant, dans l'ordre, les étapes de :
(i) application d'une composition selon l'une quelconque des revendications 1 à 6 sur le cuir chevelu ;
(ii) élimination par rinçage de ladite composition avec de l'eau, si nécessaire, et
(iii) laisser le cuir chevelu sécher.

8. Utilisation non-thérapeutique d'un polymorphe de carbonate de calcium pour stimuler la croissance des cheveux.
